# EUROPÄISCHE PATENTSCHRIFT

(11) **EP 0 992 474 B1**
(45) Veröffentlichungstag und Bekanntmachung des Hinweises auf die Patenterteilung: **09.04.2003**
(21) Anmeldenummer: 99118371.6
(22) Anmeldetag: 16.09.1999
(51) Int. Cl.: C07B 41/02, C07B 41/06, C07B 43/04, C07C 29/40

(54) **Verfahren zur Übertragung von alpha, beta-ungesättigten Alkylgruppen auf Elektrophile**
Process for transferring alpha,beta-unsaturated alkyl groups to electrophiles
Procédé pour le transfert de groupes alkyles alpha,béta-insaturés sur des électrophiles

(30) Priorität: 09.10.1998 DE 19846497
(43) Veröffentlichungstag der Anmeldung: 12.04.2000
(73) Patentinhaber: Chemetall GmbH, 60487 Frankfurt am Main (DE)
(72) Erfinder: Jones, Philip, Dr., Merck Sharp and Dohme, Harlow, Essex CM20 8QR (GB); Knochel, Paul, Professor Dr., 35037 Marburg (DE)
(74) Vertreter: Uppena, Franz, Dr.

(56) Entgegenhaltungen:
- J. NOKAMI: "New concept in allylation of aldehydes: regiospecific allylation of aldehydes by an allyl-transfer reaction of homoallylic alcohols" JOURNAL OF THE AMERICAN CHEMICAL SOCIETY, Bd. 120, Nr. 26, 8.Juli 1998, DC US, Seiten 6609-6610, XP002101392
- F. BARBOT: "SYNTHESE D'ALCOOLS beta-ETHYLENIQUES POSSEDANT UN MOTIF ISOPRENIQUE A PARTIR D'ORGANOMETALLIQUES DE TYPE PRENYLIQUE (CH3)2C=CHCH2M" TETRAHEDRON LETTERS, Nr. 44, 1975, OXFORD GB, Seiten 3829-3832, XP002101393
- P. MIGINIAC: "Transposition d'un alcool beta-éthylénique au sein d'un organozincique alpha-éthylénique" BULLETIN DE LA SOCIETE CHIMIQUE DE FRANCE, Nr. 11, 1968, PARIS FR, Seiten 4675-4676, XP002101394
- P. JONES: "Preparation and reactions of masked allylic organozinc reagents" JOURNAL OF ORGANIC CHEMISTRY, Bd. 64, Nr. 1, 8.Januar 1999, EASTON US, Seiten 186-195, XP002125154

## Beschreibung

Die vorliegende Erfindung betrifft ein Verfahren zur Übertragung von α,β-ungesättigten Alkylgruppen (das sind der Allylrest oder substituierte Allylreste oder substituierte und unsubstituierte Benzylverbindungen) auf elektrophile organische Verbindungen, die nachfolgend als Elektrophile bezeichnet werden.

Die Übertragung von Allylgruppen und verwandter Reste auf ein organisches Molekül mittels organometallischer Reagenzien ist eine seit langem bekannte Synthesemethode. Es werden dafür beispielsweise α,β-ungesättigte Alkylverbindungen des Magnesiums, Lithiums oder Zinks eingesetzt. Diese werden im allgemeinen durch Umsetzung eines Metalls mit einer ungesättigten Organohalogenverbindung dargestellt. Bei dieser Synthese bildet sich in einer Nebenreaktion durch sogenannte "Wurtz-Kupplung" ein gewisser Anteil Homokupplungsprodukt:

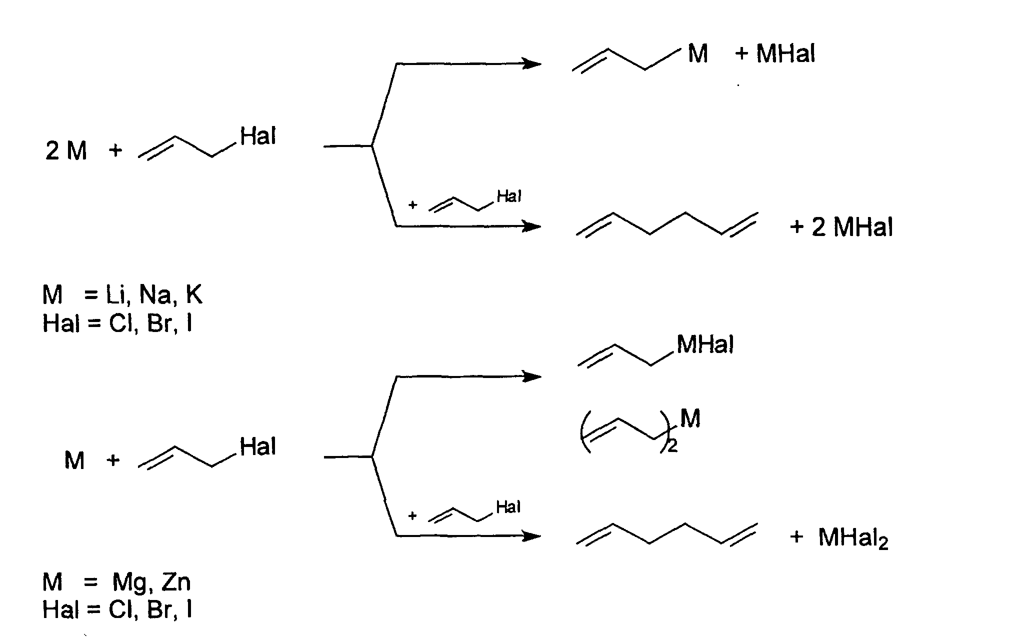

Dadurch wird die Ausbeute vermindert und die Aufarbeitung und Reinigung des gewünschten Produktes erschwert.

Der Anteil des Homokupplungsproduktes (ein 1,5-Dien) ist von der Natur des Metalls und von den spezifischen Reaktionsbedingungen abhängig. Im allgemeinen führt eine Absenkung der Reaktionstemperaturen zu einer verbesserten Organometallausbeute. Tiefe Temperaturen verlangsamen jedoch generell die Reaktionsgeschwindigkeit und sind, da sie nur durch hohen Energieverbrauch aufrechterhalten werden können, selten wirtschaftlich.

Ein besonderes Problem stellt die Übertragung in Allylposition substituierter Verbindungen auf ein Elektrophil dar. In diesem Falle tritt in der Regel eine ungewünschte Umlagerung unter Bildung des thermodynamisch stabileren, in der Olefinposition substituierten Isomers ein, so daß Produktgemische oder nur das Isomerisierungsfolgeprodukt erhalten werden:

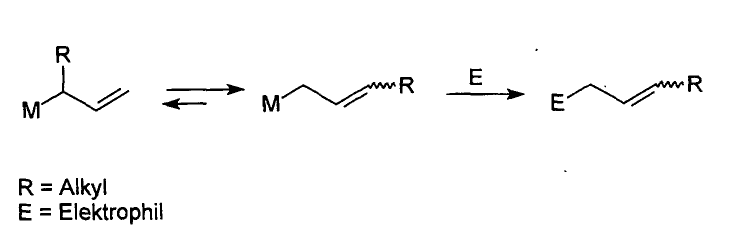

Bei der "anionischen" Übertragung substituierter Allylgruppen auf ein Elektrophil werden in der Regel schlechte Regioselektivitäten beobachtet. So wird z.B. bei der Umsetzung von E-2-butenylzinkbromid mit Benzaldehyd ein 1:1-Gemisch der beiden Diastereoisomeren erhalten.

Nokami et al. (J.Am.Chem.Soc.1998,120,6609-6610) beschreiben die Übertragung von Allylverbindungen auf einen Aldehyd in Gegenwart einer katalytischen Menge Sn(OTf)₂ (Tf = Triflat).

Aufgabe der Erfindung ist es, die Nachteile des Standes der Technik zu beseitigen und ein Verfahren zu schaffen, das es erlaubt, α,β-ungesättigte Alkylgruppen ohne Isomerisierung stereoselektiv auf ein Elektrophil zu übertragen.

Die Aufgabe wird durch das in Anspruch 1 angegebene Verfahren gelöst. Die Ansprüche 2 bis 8 bilden das angegebene Verfahren weiter.

Zur Übertragung einer α,β-ungesättigten Alkylgruppe (A) 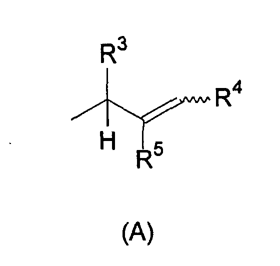 wird ein sterisch anspruchsvoll substituiertes Zinkenolat (B) (d.h. R¹ und R² sind voluminöse Substituenten), bzw. das mit diesem Zinkenolat im Gleichgewicht stehende Zinkalkyl (C) verwendet, das mit einem Elektrophil (E) zum gewünschten Produkt (D) umgesetzt wird:

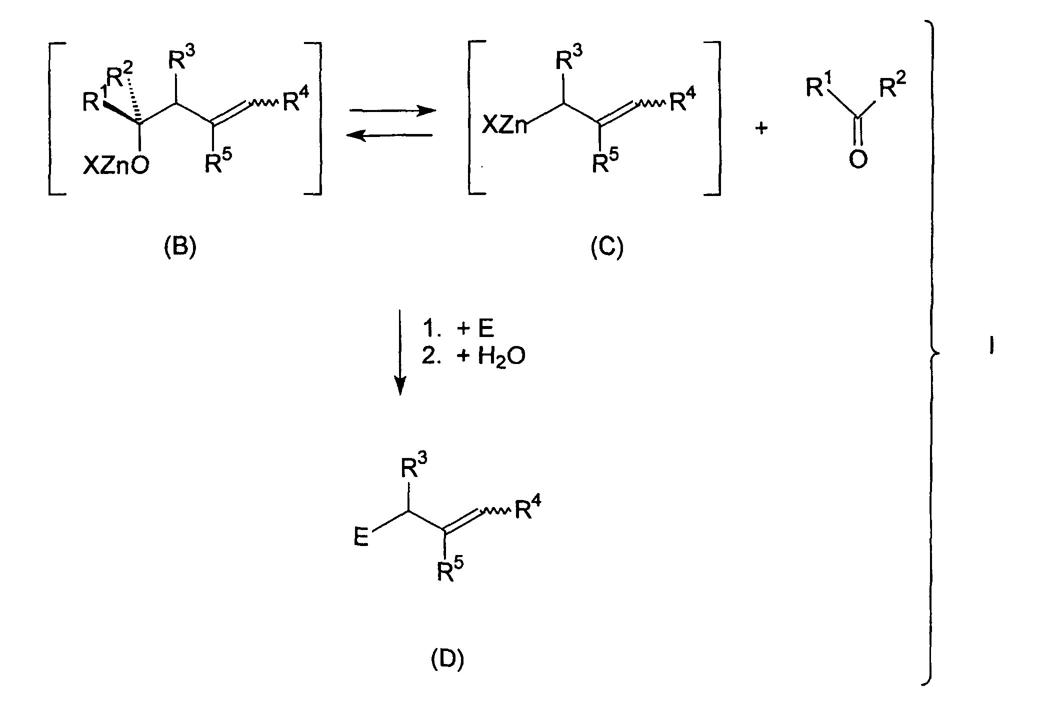

Dabei ist 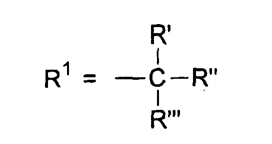
mit R' = H, Alkyl und R" = Alkyl und R''' = Alkyl,
R² = R¹ oder Phenyl,
R³, R⁴ = unabhängig voneinander H, Alkyl, Aryl, Heteroaryl,
R⁵ = H, Alkyl, Aryl, eine funktionelle Gruppe (z.B. Ether, Ester, Nitril, C=C Doppelbindung),
R⁴ und R⁵ oder R³ und R⁵ können über einen Ringschluß miteinander verbunden sein,
X = Cl, Br, I, Alkyl oder O-C(R¹,R²)-A und
E = Elektrophil = Aldehyd, Keton, Nitril, Imin, Alkin.

Der Erfindung liegt die überraschende Beobachtung zugrunde, daß sterisch anspruchsvolle Zinkenolate vom Typ (B) im Gegensatz zu den Enolaten anderer Metalle nicht stabil sind, sondern unter Bildung eines sterisch anspruchsvollen Ketons und einer Allylzinkverbindung (C) fragmentieren. Das System (B,C), im folgenden "maskiertes Zinkalkyl" genannt, kann mit einem Elektrophil (E) unter Bildung eines wertvollen Produktes (D) reagieren.

Das maskierte Zinkalkyl (B,C) kann durch Ummetallierung einer die α,β-ungesättigte Alkylgruppe (A) enthaltenden Verbindung (F) gemäß folgender Gleichung hergestellt werden: 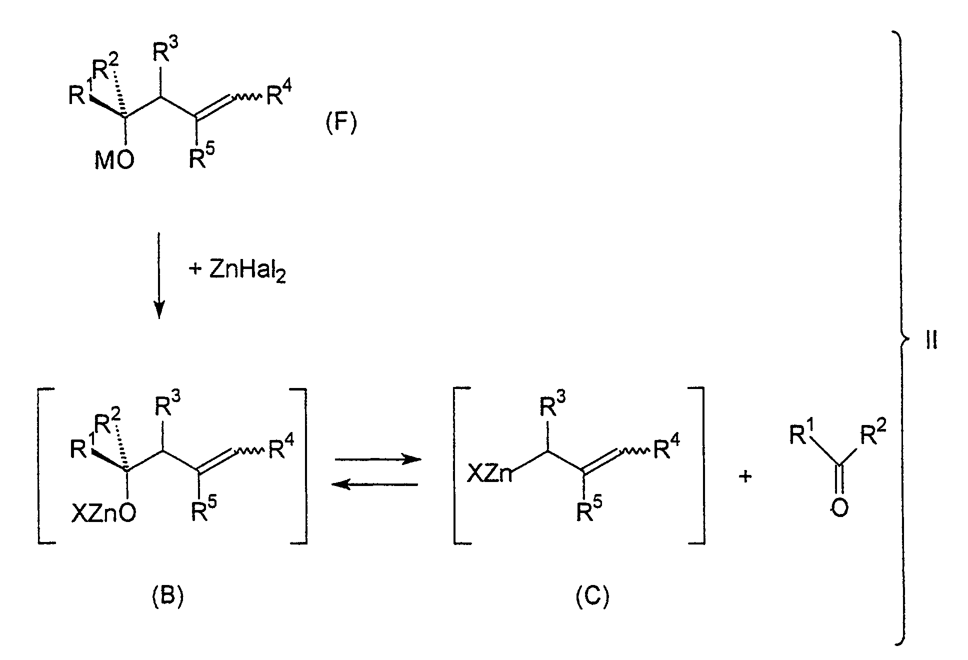
- M =: Li, Na, K, MgX und
- Hal =: Cl, Br, I

Das maskierte Zinkalkyl (B,C) wird im Regelfall nicht isoliert, sondern in situ gebildet und gleich mit dem Elektrophil (E) umgesetzt.

Zweckmäßigerweise ist dabei das Molverhältnis des eingesetzten Metallenolates (F) zum Elektrophil (E) weitgehend stöchiometrisch (1 : (0,8 bis 3,0), bevorzugt 1 : (1,0 bis 1,2)), während das Zinksalz ZnHal₂ in einer Menge von 0,1 bis 1,5 mol, bezogen auf 1 mol der Ausgangsverbindung (F), eingesetzt werden kann. Bevorzugt ist eine Menge von 0,1 bis 0,5 mol.

Das Metallenolat (F) kann z.B. in bekannter Weise wie folgt hergestellt werden:

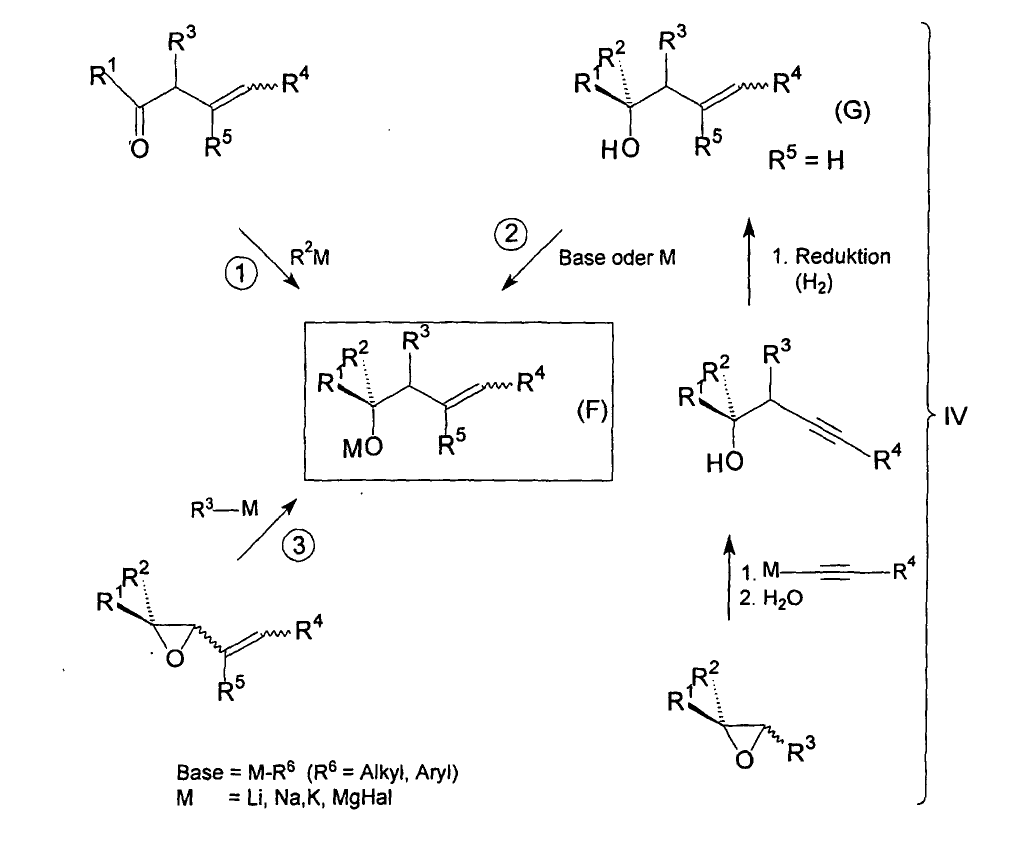

Der für den Einzelfall günstigste Zugang zum Metallenolat (F) ergibt sich unter anderem aus der individuellen Verfügbarkeit der Vorstufen.

Das maskierte Zinkalkyl (B,C) kann auch durch Metallierung einer die α,β-ungesättigte Alkylgruppe (A) enthaltenden Verbindung (G) mittels einer Dialkylzinkverbindung ZnR₂ gemäß folgender Gleichung hergestellt werden: 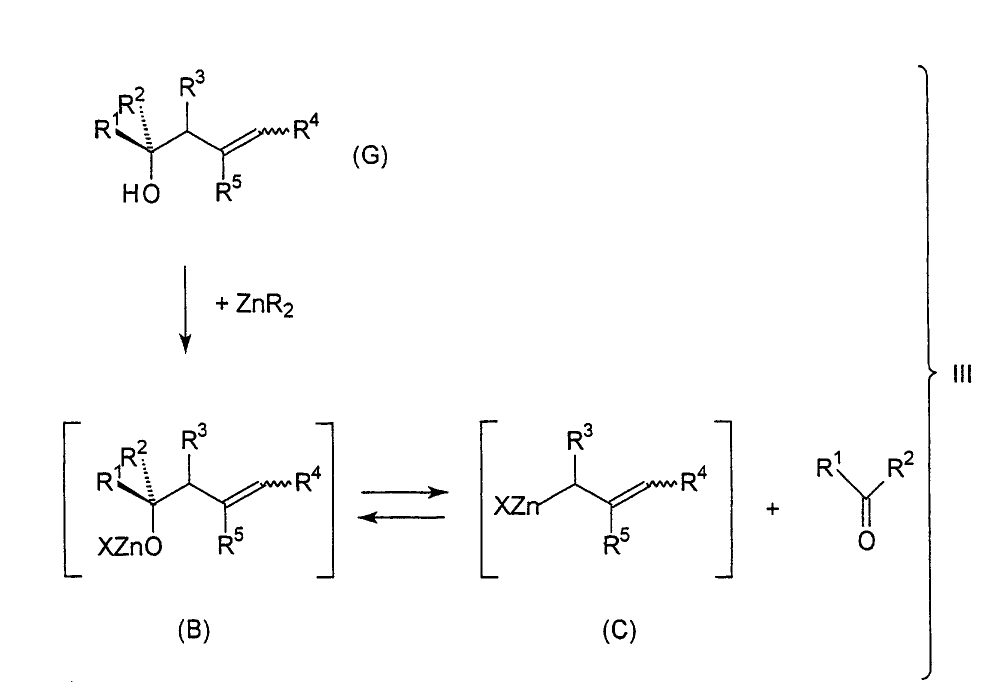
- R =: Alkyl mit 1 bis 6 C-Atomen

Gut läßt sich diese Reaktion z.B. mit Diethylzink durchführen.

Auch bei dieser Verfahrensvariante wird das maskierte Zinkalkyl (B,C) im Regelfall nicht isoliert, sondern in situ gebildet und gleich mit dem Elektrophil (E) umgesetzt.

Alle bisher beschriebenen Reaktionen werden in einem aprotischen Lösungsmittel durchgeführt. Als aprotisches Lösungsmittel können Ether, Amide oder Kohlenwasserstoffe oder Gemische dieser Lösungsmittel verwendet werden. Als Ether können beispielsweise Diethylether, Tetrahydrofuran (THF) oder 2-Methyl-THF, als Amid können beispielsweise Hexamethylphosphorsäuretriamid (HMPT), N-Methyl-2-pyrrolidinon (NMP), N,N-Dimethylformamid (DMF) oder 1,3-Dimethyltetrahydro-2(1H)-pyrimidinon (DMPU) und als Kohlenwasserstoff können beispielsweise Toluol, Hexan, Heptan oder Cyclohexan eingesetzt werden.

Es wurde beobachtet, daß die Geschwindigkeit der Fragmentierung (B)->(C) durch die Donorstärke des Lösungsmittels beeinflußt wird. Je polarer das Lösungsmittel ist, desto schneller erfolgt die Spaltung, d.h. desto mehr kann die Reaktionstemperatur gesenkt werden. Der zweite Faktor, der die Geschwindigkeit der Fragmentierung bestimmt, ist das spezifische Substitutionsmuster des Zinkenolates (B). Die folgende Tabelle 1 gibt den jeweils empfohlenen Temperaturbereich für die Allylübertragungsreaktion (I) an:

**Tabelle 1**

| lfd. Nr. | Lösungsmittel | Substituent | | | | | typische Reaktionstemp. (°C) | typische Reaktionszeit |
|---|---|---|---|---|---|---|---|---|
| | | R¹ | R² | R³ | R⁴ | R⁵ | | |
| 1 | THF/Hexan | ^{t}Bu | ^{t}Bu | H | H | H | 0 bis 40 | Min -2 Std. |
| 2 | THF/Hexan | ⁱPr | ⁱPr | H | H | H | 40 bis 100 | 10 Std. |
| 3 | THF/HMPT | ⁱPr | ⁱPr | H | H | H | 40 bis 100 | 5 Std. |
| 4 | THF/Hexan | ^{t}Bu | ^{t}Bu | Alkyl | H | H | -80 bis -20 | Min |
| 5 | THF/Hexan | ^{t}Bu | ^{t}Bu | H | Cyclo | C4H₄* | 40 bis 100 | 5 Std. |
| 6 | THF | ^{t}Bu | ^{t}Bu | H | Alkyl | H | 20 bis 100 | einige Std. |
| 7 | THF | ^{t}Bu | ^{t}Bu | H | H | Alkyl | 0 bis 40 | einige Std. |

| | | | | | | | | |
|---|---|---|---|---|---|---|---|---|
| * = Benzyl (R³ und R⁴ sind über einen Ringschluß verbunden), | | | | | | | | |
| ^{t}Bu = tert-Butyl, | | | | | | | | |
| ⁱPr = iso-Propyl | | | | | | | | |

Erfolgt die Herstellung des maskierten Zinkalkyls (B,C) aus der Alkoholvorstufe gemäß Reaktion (III), gibt es bezüglich des Restes R prinzipiell keine Beschränkungen, jedoch sind die reaktiveren Zinkverbindungen mit kurzkettigen Resten (C₁ bis C₄) bevorzugt. Ganz besonders bietet sich Diethylzink an, das kommerziell erhältlich ist. Die Umsetzung erfolgt in stark koordinierenden Solvenzien (z.B. einer Mischung aus THF und DMPU) im Temperaturbereich zwischen ca. 0 und 70°C in Gegenwart des Elektrophils. Wegen der geringen Reaktivität von Dialkylzinkverbindungen gegenüber Alkoholen benötigt die Reaktion einige bis viele Stunden.

Demgegenüber ist der Metallaustausch bei der Umsetzung von Metallenolaten (F) mit Zinkhalogeniden gemäß Reaktion (II) wesentlich schneller, so daß selbst bei tiefen Temperaturen (z.B. -78°C) kurze Reaktionszeiten beobachtet werden. Diese Variante ist im Vergleich zu der oben beschriebenen Bildungsweise aus Alkohol und Dialkylzinkverbindung bevorzugt. Dies gilt besonders für solche α,β-ungesättigten Alkylgruppen (A) und/oder Elektrophile (E), die bei höheren Temperaturen in unerwünschter Weise isomerisieren können.

Der Metall/Zinkaustausch (II) kann demzufolge bei Temperaturen, die an die jeweiligen Strukturen angepaßt sind, vorgenommen werden. Der Austausch findet bevorzugt in Gegenwart des elektrophilen Empfängermoleküls (E) unter Beachtung der in obiger Tabelle angegebenen Reaktionstemperaturen statt. Im Falle isomerisierungsstabiler α,β-ungesättigeter Alkylgruppen (A) kann das Elektrophil E auch erst nach Beendigung des Metall/Zinkaustausches zugegeben werden. Für diesen Fall verlängern sich jedoch die benötigten Reaktionszeiten entsprechend.

Ein weiterer Vorteil des erfindungsgemäßen Verfahrens ist, daß substituierte Allylgruppen (A) (R³ ≠ H) bei genügend tiefen Temperaturen (z.B. -78 °C) mit sehr guten Diastereoselektivitäten übertragen werden können und auch bei Raumtemperatur keine Isomerisierung auftritt, während bei der direkten, nicht erfindungsgemäßen Addition von Allylzinkhalogeniden ein Anti:Syn-Verhältnis von etwa 50:50 erhalten wird.

Der Gegenstand der Erfindung wird nachstehend anhand von Ausführungsbeispielen näher erläutert.

### Beispiel 1:

Herstellung von 1-Phenyl-but-3-en-1-ol durch Übertragung der 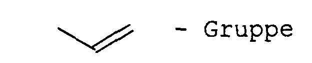 auf Benzaldehyd mit R¹ = R² = ^{t}Bu
1,94 ml einer 1,4 molaren Lösung von n-Butyllithium (2,71 mmol) in Pentan wurden bei 0 °C unter Argon während 2 Min zu einer gerührten Lösung von 500 mg ( 2,71 mmol) 3-tert-Butyl-2,2-dimethylhex-5-en-3-ol in 4 ml THF zugetropft. Die erhaltene Lösung wurde 15 Min gerührt und dann eine Lösung von 610 mg (2,71 mmol) ZnBr₂ in 2 ml THF und anschließend 275 µl (2,71 mmol) Benzaldehyd zugegeben. Das Reaktionsgemisch wurde 1 Std. gerührt und auf Zimmertemperatur erwärmt. Anschließend wurde mit 15 ml NH₄Cl-Lösung versetzt. Die organischen Anteile wurden 3 mal mit je 15 ml Et₂O extrahiert, die vereinigten organischen Phasen mit 10 ml Salzwasser gewaschen, dann getrocknet und unter reduziertem Druck aufkonzentriert. Das erhaltene Rohprodukt wurde über eine Silicasäule mit 15 % Diethylether / Leichtbenzin gereinigt. Erhalten wurde der Alkohol als ein farbloses Öl.
Ausbeute: 356 mg (89 %)

### Beispiel 2:

Herstellung von 1-Phenyl-but-3-en-1-ol durch Übertragung der 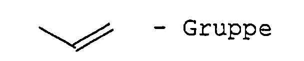 auf Benzaldehyd mit R¹ = R² = tBu unter Verwendung einer unterstöchiometrischen Menge ZnBr₂

Die Reaktion wurde anolog Beispiel 1 mit 61 mg ( 0,27 mmol) ZnBr₂ durchgeführt. Erhalten wurde der Alkohol als ein farbloses Öl.
Ausbeute: 368 mg (92 %)

### Beispiel 3:

Herstellung von 1-Phenyl-but-3-en-1-ol durch Übertragung der 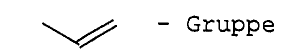 auf Benzaldehyd mit R¹ = R² = ⁱPr
2,29 ml einer 1,4 molaren Lösung von n-Butyllithium (3,20 mmol) in Pentan wurden bei 0 °C unter Argon während 2 Min zu einer gerührten Lösung von 500 mg ( 3,20 mmol) 2-Methyl-3-isopropyl-hex-5-en-3-ol in 2 ml THF zugetropft. Die erhaltene Lösung wurde auf Raumtemperatur erwärmt, 15 Min gerührt und dann eine Lösung von 721 mg (3,20 mmol) ZnBr₂ in 2 ml THF und anschließend 330 µl (3,20 mmol) Benzaldehyd und 6 ml HMPA zugegeben. Das Reaktionsgemisch wurde auf 70 °C erwärmt und 6 Std. bei dieser Temperatur gerührt. Anschließend wurde auf Raumtemperatur abgekühlt und analog Beispiel 1 aufgearbeitet. Das erhaltene Rohprodukt wurde über eine Silicasäule mit 15 % Diethylether / Leichtbenzin gereinigt. Erhalten wurde der Alkohol als ein farbloses Öl.
Ausbeute: 464 mg (98 %)

### Beispiel 4:

Herstellung von 1-Phenyl-but-3-en-1-on durch Übertragung der 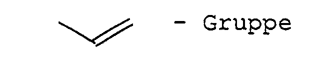 auf Benzonitril
1,94 ml einer 1,4 molaren Lösung von n-Butyllithium (2,71 mmol) in Pentan wurden bei 0 °C unter Argon während 2 Min zu einer gerührten Lösung von 500 mg ( 2,71 mmol) 3-tert-Butyl-2,2-dimethylhex-5-en-3-ol in 4 ml THF zugetropft. Die erhaltene Lösung wurde 15 Min gerührt, eine Lösung von 370 mg (2,71 mmol) ZnCl₂ in 2 ml THF und danach 278 µl (2,71 mmol) Benzonitril zugegeben. Das Reaktionsgemisch wurde auf Zimmertemperatur erwärmt und 1 Std. gerührt. Anschließend wurde mit 15 ml einer 0,25 molaren HCl-Lösung und mit 30 ml Et₂O versetzt und die erhaltene Mischung 10 Min kräftig gerührt. Die Phasen wurden getrennt, die organischen Anteile wurden 2 mal mit je 15 ml Et₂O aus der wäßrigen Phase extrahiert, die vereinigten organischen Phasen mit 10 ml Salzwasser gewaschen, dann getrocknet und unter reduziertem Druck aufkonzentriert. Das erhaltene Rohprodukt wurde über eine Silicasäule mit 5 % Diethylether / Leichtbenzin gereinigt. Erhalten wurde das Keton als ein farbloses Öl.
Ausbeute: 289 mg (73 %)

### Beispiel 5:

Herstellung von 1-Allylcyclohexanol durch Übertragung der 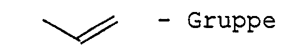 auf Cyclohexanon
1,94 ml einer 1,4 molaren Lösung von n-Butyllithium (2,71 mmol) in Pentan wurden bei 0 °C unter Argon während 2 Min zu einer gerührten Lösung von 500 mg ( 2,71 mmol) 3-tert-Butyl-2,2-dimethylhex-5-en-3-ol in 4 ml THF zugetropft. Die erhaltene Lösung wurde 15 Min gerührt und dann eine Lösung von 610 mg (2,71 mmol) ZnBr₂ in 2 ml THF und 280 µl (2,71 mmol) Cyclohexanon zugegeben. Das Reaktionsgemisch wurde 4 Std. gerührt und auf Zimmertemperatur erwärmt. Anschließend wurde analog Beispiel 1 aufgearbeitet. Das erhaltene Rohprodukt wurde über eine Silicasäule mit 20 % Diethylether / Leichtbenzin gereinigt. Erhalten wurde der Alkohol als ein farbloses Öl.
Ausbeute: 325 mg (82 %)

### Beispiel 6:

Herstellung von (1-Allyl-3-methyl-but-2-enyl)benzylamin durch Übertragung der 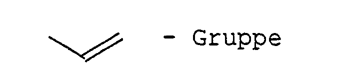 auf Benzyl-(3-methyl-but-2-enyliden)amin

Die Reaktion wurde analog Beispiel 1 mit 500 mg (2,71 mmol) 3-tert-Butyl-2,2-dimethylhex-5-en-3-ol, 1,93 ml (2,71 mmol) einer 1,4 molaren n-Butyllithiumlösung in Pentan, 468 mg (2,71 mmol) Benzyl-(3-methyl-but-2-enyliden)amin und 370 mg (2,71 mmol) ZnCl₂ durchgeführt. Das erhaltene Rohprodukt wurde über eine Silicasäule mit 25-100 % Diethylether /Leichtbenzin gereinigt. Erhalten wurde das Amin als ein farbloses Öl.
Ausbeute: 522 mg (90 %)

### Beispiel 7:

Herstellung von *Threo* 2-Methyl-1-phenyl-3-buten-1-ol durch stereoselektive Übertragung der 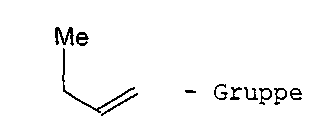 auf Benzaldehyd
1,58 ml einer 1,6 molaren Lösung von n-Butyllithium (2,52 mmol) in Pentan wurden bei -78 °C unter Argon während 5 Min zu einer gerührten Lösung von 500 mg ( 2,52 mmol) 3-tert-Butyl-2,2,4-trimethylhex-5-en-3-ol in 4 ml THF zugetropft. Die erhaltene Lösung wurde 15 Min gerührt, dann 256 µl (2,52 mmol) Benzaldehyd und anschließend während 3 Min eine Lösung von 343 mg (2,52 mmol) ZnCl₂ in 2 ml THF zugegeben. Das Reaktionsgemisch wurde 1 Std. bei -78 °C gerührt und dann auf Zimmertemperatur erwärmt. Anschließend wurde analog Beispiel 1 aufgearbeitet. Das erhaltene Rohprodukt wurde über eine Silicasäule mit 10 % Diethylether / Leichtbenzin gereinigt. Erhalten wurde der Alkohol als ein blaßgelbes Öl.
Ausbeute: 341 mg (83 %), Anti:Syn-Verhältnis 94:6

### Beispiel 8:

Herstellung von *cis* und *trans* 1-Phenyl-3-hepten-1-ol ausgehend von 2,2-di-tert-Butyloxiran
1,26 ml (12,8 mmol) 1-Pentin wurden bei 0 °C unter Argon während 5 Min zu 8,0 ml (12,8 mmol) einer 1,6 molaren n-Butyllithiumlösung in Pentan tropfenweise zugegeben. Die erhaltene Lösung wurde weitere 15 Min gerührt und dann das Lösungsmittel unter vermindertem Druck abgezogen. Es wurden 5 ml HMPA und eine Lösung von 1,0 g ( 6,4 mmol) 2,2-di-tert-Butyloxiran in 2 ml HMPA zugegeben. Die Reaktionslösung wurde 20 Std. bei Raumtemperatur gerührt. Anschließend wurde analog Beispiel 1 aufgearbeitet. Das erhaltene Rohprodukt wurde über eine Silicasäule mit 2 % Diethylether /Leichtbenzin gereinigt. Erhalten wurden 1,21 g (84 %) 3-tert-Butyl-2,2-dimethylnon-5-in-3-ol 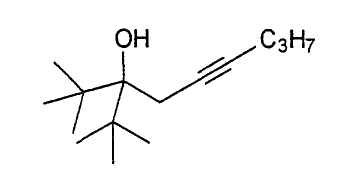 als ein farbloses Öl.

50 mg eines Katalysators aus 5 % Palladium auf Bariumsulfat wurden auf einmal zu einer gerührten Lösung von 500 mg (2,23 mmol) 3-tert-Butyl-2,2-dimethylnon-5-in-3-ol in 5 ml Pyridin gegeben. Die erhaltene Mischung wurde 3 mal entgast, einer Wasserstoffatmosphäre ausgesetzt und über Nacht gerührt. Anschließend wurde mit 100 ml Pentan verdünnt und über Silicagel filtriert. Das Silicagel wurde mit 100 ml Et₂O gewaschen. Die vereinigten organischen Phasen wurden mit einer Kupfersulfatlösung (5 x 100 ml), mit H₂O (100 ml) und mit Salzlösung (50 ml) gewaschen, getrocknet und unter reduziertem Druck konzentriert. Erhalten wurden 503 mg (99%) (Z)-3-tert-Butyl-2,2-dimethylnon-5-en-3-ol 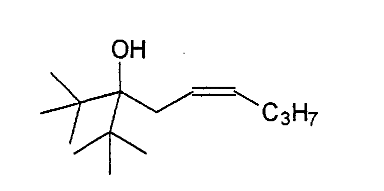 als ein farbloses Öl.

Die weitere Reaktion wurde analog Beispiel 7 mit 452 mg (2,0 mmol) (Z)-3-tert-Butyl-2,2-dimethylnon-5-en-3-ol, 1,45 ml (2,0 mmol) einer 1,38 molaren n-Butyllithiumlösung in Pentan, 202 µl (2,0 mmol) Benzaldehyd und 272 mg ( 2,0 mmol) ZnCl₂ durchgeführt. Nach Erwärmung auf Raumtemperatur wurde die Reaktionslösung 80 Std. gerührt. Das erhaltene Rohprodukt wurde über eine Silicasäule mit 2-10 % Diethylether / Leichtbenzin gereinigt. Dabei wurden zuerst 180 mg (40 %) des Eduktes und dann 197 mg (52 %) des Produktes 1-Phenyl-3-hepten-1-ol 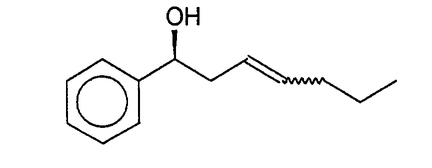 (Gemisch der *cis* und *trans* Isomeren) als farbloses Öl erhalten.

### Beispiel 9:

### Herstellung von 1,2-Diphenyl-1-ethanol

1,33 ml einer 1,6 molaren Lösung von n-Butyllithium (2,13 mmol) in Pentan wurden bei Raumtemperatur unter Argon während 2 Min zu einer gerührten Lösung von 500 mg ( 2,13 mmol) 3-Benzyl-2,2,4,4-tetramethyl-3-pentanol in 4 ml THF zugetropft. Die erhaltene Lösung wurde 30 Min gerührt, dann 275 µl (2,13 mmol) Benzaldehyd, 290 mg (2,13 mmol) ZnCl₂ und 2 ml HMPA zugegeben. Das Reaktionsgemisch wurde auf 70 °C erwärmt und 4 Std. bei dieser Temperatur gerührt. Anschließend wurde auf Raumtemperatur abgekühlt und analog Beispiel 1 aufgearbeitet. Das erhaltene Rohprodukt wurde über eine Silicasäule mit 35 % Diethylether / Leichtbenzin gereinigt. Erhalten wurde der Alkohol als ein farbloses Öl.
Ausbeute: 327 mg (78 %)

### Beispiel 10:

### Herstellung von 2-Methylen-pent-4-en-1-ol

3,9 ml einer 1,4 molaren Lösung von n-Butyllithium (5,4 mmol) in Pentan wurden bei 0 °C unter Argon während 2 Min zu einer gerührten Lösung von 1,0 g ( 5,4 mmol) 3-tert-Butyl-2,2-dimethylhex-5-en-3-ol in 8 ml THF zugetropft. Die erhaltene Lösung wurde 15 Min gerührt, dann 741 mg (5,4 mmol) ZnCl₂ und 347 mg (2,71 mmol) Trimethylprop-2-inyloxysilan zugegeben. Das Reaktionsgemisch wurde auf Raumtemperatur erwärmt, 2 Std. gerührt, in 10 ml einer 2 molaren HCl-Lösung und 10 ml Et₂O gegossen, weitere 15 Min gerührt und analog Beispiel 1 aufgearbeitet. Das erhaltene Rohprodukt wurde über eine Silicasäule mit 25 % Diethylether / Leichtbenzin gereinigt. Erhalten wurde der Alkohol als ein farbloses Öl.
Ausbeute: 202 mg (74 %)

## Patentansprüche

1. Verfahren zur Übertragung von α,β-ungesättigten Alkylgruppen (A) 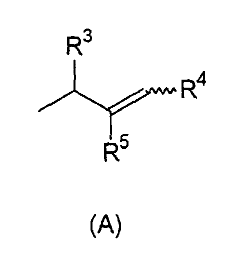 auf ein Elektrophil (E), **dadurch gekennzeichnet, daß** die α,β-ungesättigte Alkylgruppe (A) mittels eines maskierten Zinkalkyls (B,C) nach der allgemeinen Gleichung I 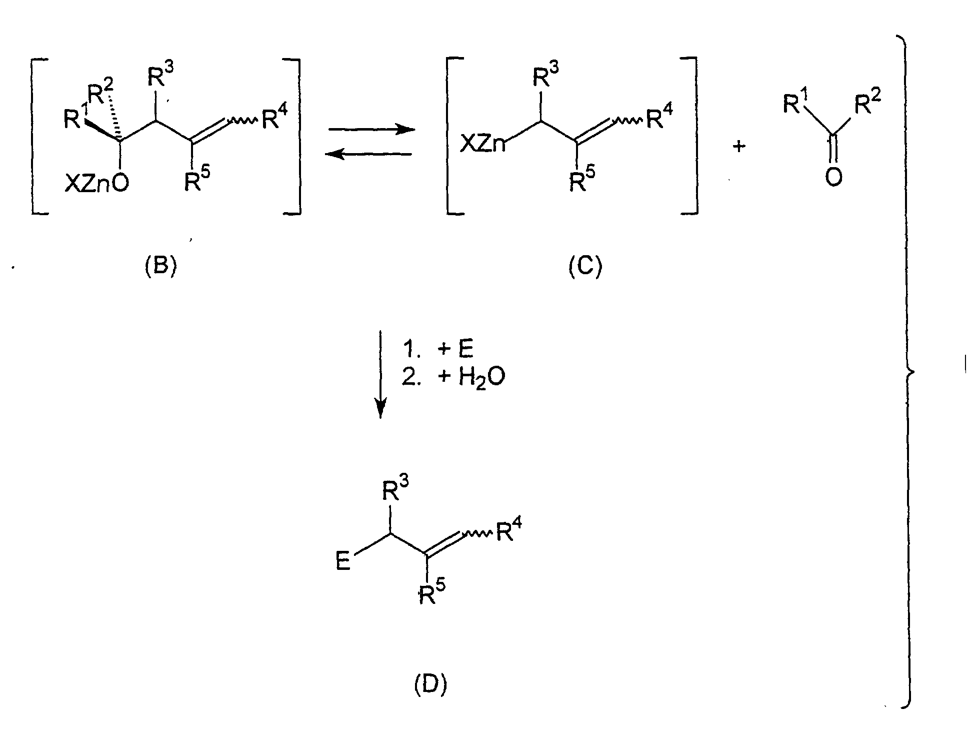 auf das Elektrophil (E) übertragen wird, wobei 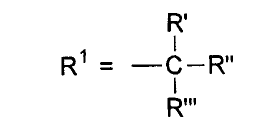
mit R' = H, Alkyl und R" = Alkyl und R''' = Alkyl,
R² = R¹ oder Phenyl,
R³, R⁴ = unabhängig voneinander H, Alkyl, Aryl, Heteroaryl,
R⁵ = H, Alkyl, Aryl, eine funktionelle Gruppe (z.B. Ether, Ester, Nitril, C=C Doppelbindung) ist,
R⁴ und R⁵ oder R³ und R⁵ über einen Ringschluß miteinander verbunden sein können,
X = Cl, Br, I, Alkyl oder O-C(R¹,R²)-A und
E = Elektrophil = Aldehyd, Keton, Nitril, Imin, Alkin ist und die Reaktion in einem aprotischen Lösungsmittel durchgeführt wird.

2. Verfahren nach Anspruch 1, **dadurch gekennzeichnet, daß** das maskierte Zinkalkyl (B,C) hergestellt wird durch Ummetallierung einer die α,β-ungesättigte Alkylgruppe (A) enthaltenden Verbindung (F) nach der allgemeinen Gleichung II 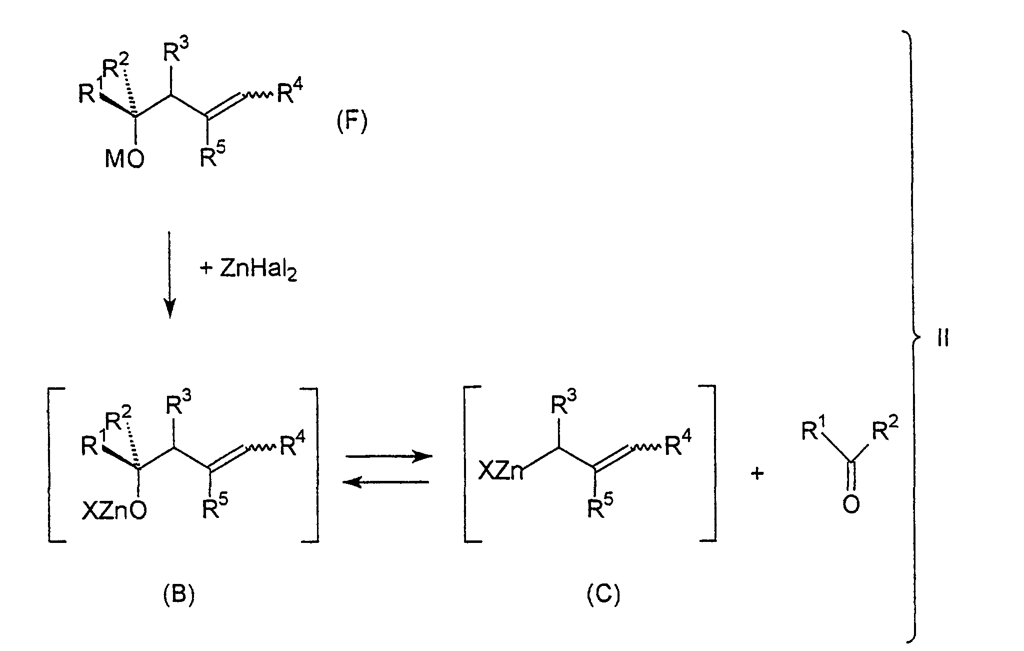 wobei
M = Li, Na, K, MgX und
Hal = Cl, Br, I
ist und die Reaktion in einem aprotischen Lösungsmittel durchgeführt wird.

3. Verfahren nach Anspruch 2, **dadurch gekennzeichnet, daß** die Verbindung (F), das Zinksalz ZnHal₂ und das Elektrophil (E) im Molverhältnis 1 : (0,1 bis 1,5) : (0,8 bis 3,0) eingesetzt werden.

4. Verfahren nach einem der Ansprüche 2 bis 3, **dadurch gekennzeichnet, daß** das Zinksalz ZnHal₂ in einer Menge von 0,1 bis 0,5 mol, bezogen auf 1 mol der Ausgangsverbindung F, eingesetzt wird.

5. Verfahren nach Anspruch 1, **dadurch gekennzeichnet, daß** das maskierte Zinkalkyl (B,C) hergestellt wird durch Metallierung einer die α,β-ungesättigte Alkylgruppe (A) enthaltenden Verbindung (G) mittels einer Dialkylzinkverbindung ZnR₂ nach der allgemeinen Gleichung III 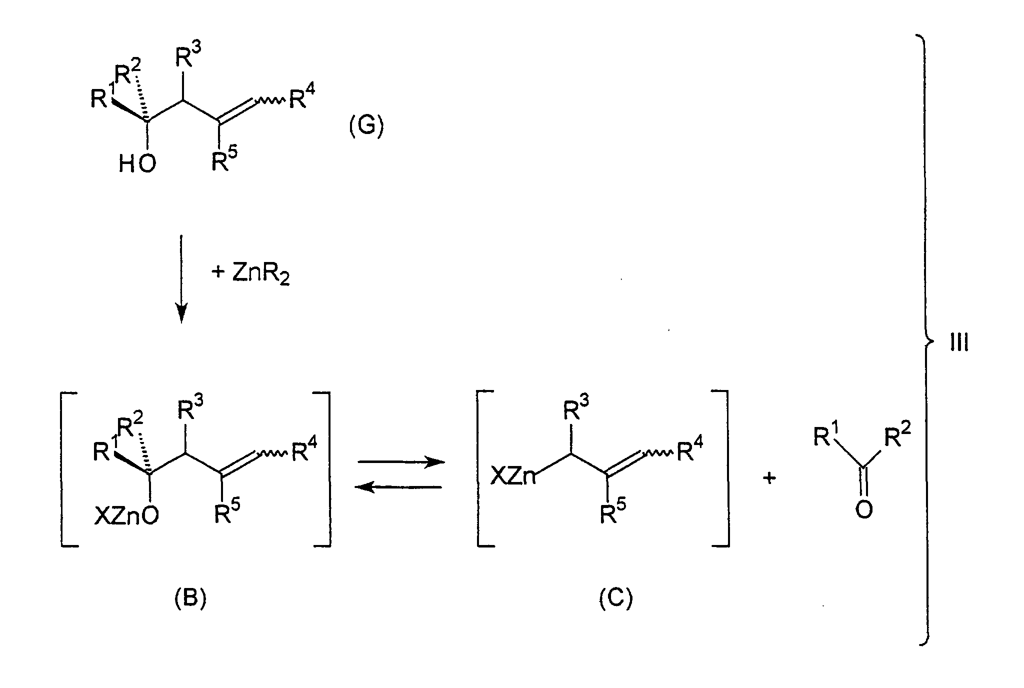 wobei
R = Alkyl mit 1 bis 6 C-Atomen
ist und die Reaktion in einem aprotischen Lösungsmittel durchgeführt wird.

6. Verfahren nach einem der Ansprüche 1 bis 5, **dadurch gekennzeichnet, daß** die Herstellung des maskierten Zinkalkyls (B,C) und die Umsetzung mit dem Elektrophil (E) als Eintopfreaktion durchgeführt werden.

7. Verfahren nach einem der Ansprüche 1 bis 6, **dadurch gekennzeichnet, daß** als aprotisches Lösungsmittel Ether, Amide oder Kohlenwasserstoffe oder Gemische dieser Lösungsmittel verwendet werden.

8. Verfahren nach einem der Ansprüche 1 bis 7, **dadurch gekennzeichnet, daß** als aprotisches Lösungsmittel Diethylether, Tetrahydrofuran (THF), 2-Methyl-THF, Hexamethylphosphorsäuretriamid (HMPT), N-Methyl-2-pyrrolidinon (NMP), N,N-Dimethylformamid (DMF), 1,3-Dimethyltetrahydro-2(1H)-pyrimidinon (DMPU), Toluol, Hexan, Heptan, Cyclohexan oder Gemische dieser Lösungsmittel verwendet werden.

## Claims

1. Method for transferring α,β-unsaturated alkyl groups (A) 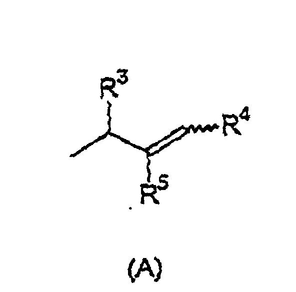 onto an electrophile (E), **characterised in that** the α,β-unsaturated alkyl group (A) is transferred onto the electrophile (E) by means of a masked zinc alkyl (B, C) in accordance with the general equation I 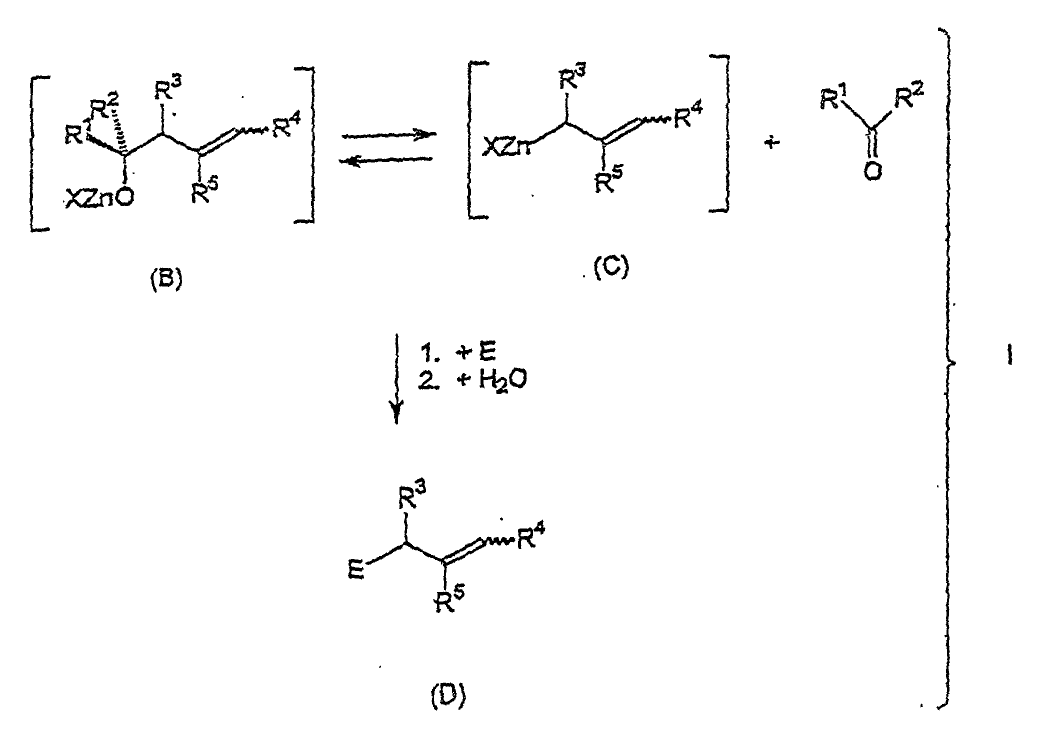 where 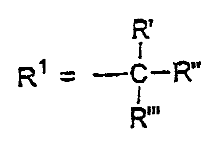
with R' = H, alkyl and R'' = alkyl and R''' = alkyl,
R² = R¹ or phenyl,
R³, R⁴ = independently of each other, H, alkyl, aryl, heteroaryl,
R⁵ = H, alkyl, aryl, a functional group (for example ether, ester, nitrile, C=C double bond),
R⁴ and R⁵ or R³ and R⁵ can be connected together by way of a ring closure,
X = Cl, Br, I, alkyl or O-C(R¹,R²)-A and
E = electrophile = aldehyde, ketone, nitrile, imine, alkyne and the reaction is carried out in an aprotic solvent.

2. Method according to claim 1, **characterised in that** the masked zinc alkyl (B,C) is prepared by re-metalation of a compound (F), containing the α,β-unsaturated alkyl group (A), in accordance with the general equation II 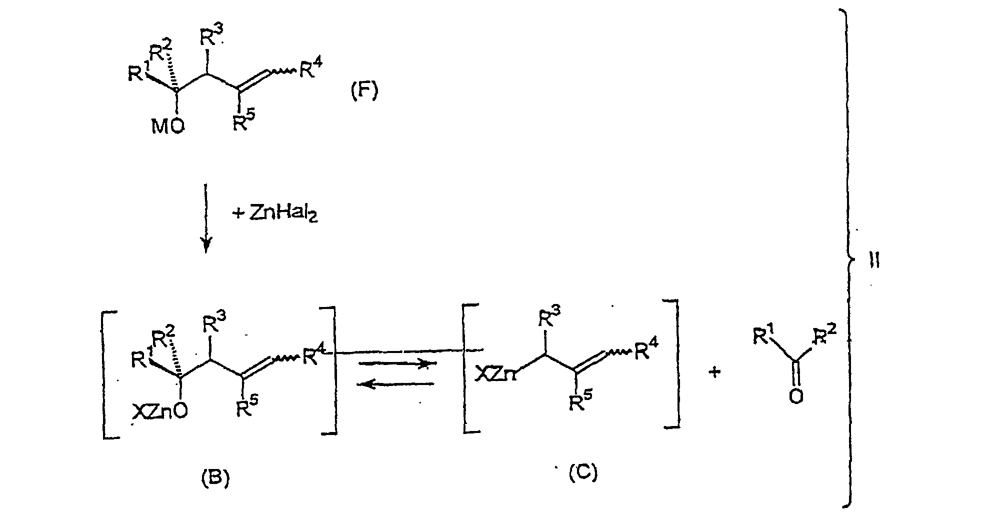 where
M = Li, Na, K, MgX and
Hal = Cl, Br, I
and the reaction is carried out in an aprotic solvent.

3. Method according to claim 2, **characterised in that** the compound (F), the zinc salt ZnHal₂ and the electrophile (E) are used in the molar ratio of 1 : (0.1 to 1.5) : (0.8 to 3.0).

4. Method according to one of claims 2 to 3, **characterised in that** the zinc salt ZnHal₂ is used in a quantity of 0.1 to 0.5 mol, relative to 1 mol of the starting compound F.

5. Method according to claim 1, **characterised in that** the masked zinc alkyl (B,C) is prepared by metalation of a compound (G), containing the α,β-unsaturated alkyl group (A), by means of a dialkyl zinc compound ZnR₂ in accordance with the general equation III 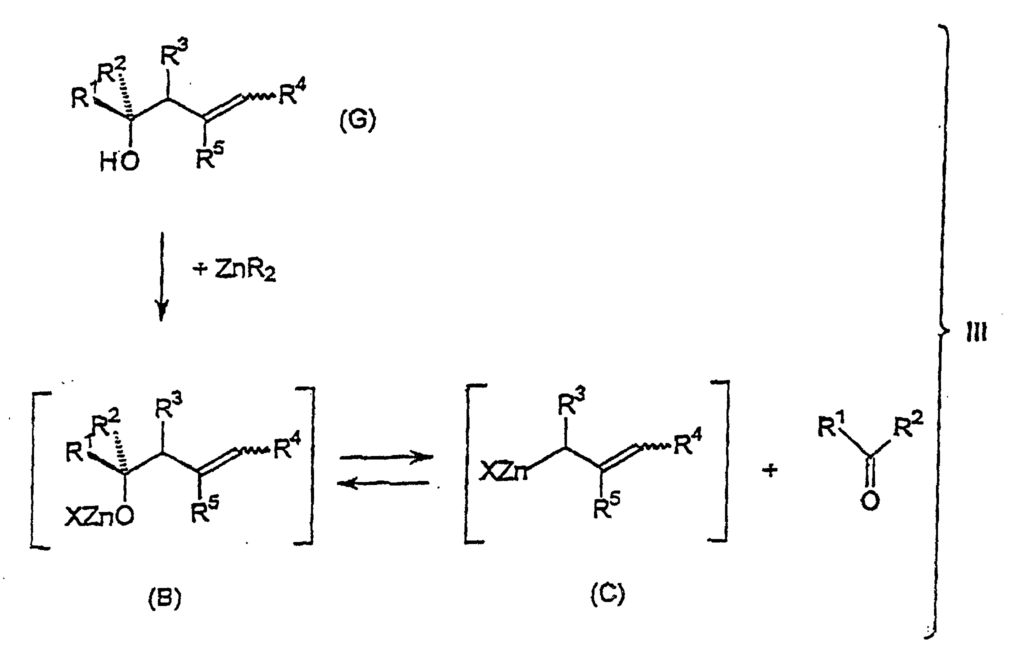 where
R = alkyl with 1 to 6 C-atoms and the reaction is carried out in an aprotic solvent.

6. Method according to one of claims 1 to 5, **characterised in that** the preparation of the masked zinc alkyl (B, C) and the conversion with the electrophile (E) are carried out as a single pot reaction.

7. Method according to one of claims 1 to 6, **characterised in that** ethers, amides or hydrocarbons or mixtures of these solvents are used as the aprotic solvent.

8. Method according to one of claims 1 to 7, **characterised in that** diethyl ether, tetrahydrofuran (THF), 2-methyl-THF, hexamethyl phosphoric triamide (HMPT), N-methyl-2-pyrrolidinone (NMP), N,N-dimethyl formamide (DMF), 1,3-dimethyltetrahydro-2(1H)-pyrimidinone (DMPU), toluene, hexane, heptane, cyclohexane or mixtures of these solvents are used as the aprotic solvent.

## Revendications

1. Procédé de transfert de groupes alkyle α,β-insaturés (A) 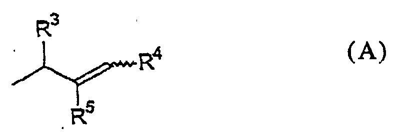 sur un électrophile (E), **caractérisé en ce que** le groupe alkyle α,β-insaturé est transféré sur l'électrophile (E) au moyen d'un alkyl-zinc masqué (B, C), d'après l'équation générale I : 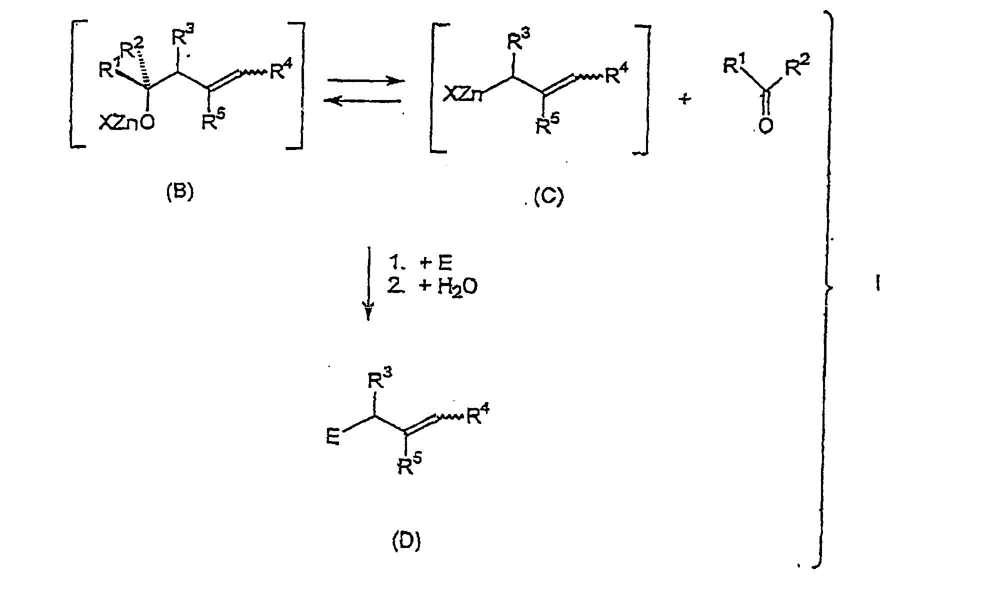 dans laquelle R¹ représente 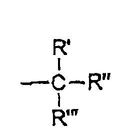
où R' représente un atome d'hydrogène ou un groupe alkyle, et R" et R''' représentent chacun un groupe alkyle,
R² a la même signification que R¹ ou représente un groupe phényle,
R³ et R⁴ représentent chacun, indépendamment l'un de l'autre, un atome d'hydrogène ou un groupe alkyle, aryle ou hétéroaryle,
R⁵ représente un atome d'hydrogène, un groupe alkyle ou aryle, ou un groupe fonctionnel (par exemple éther, ester, nitrile, double liaison C=C),
R⁴ et R⁵, ou R³ et R⁵, pouvant être raccordés l'un à l'autre en un cycle,
X représente un atome de chlore, de brome ou d'iode, un groupe alkyle ou un fragment de formule -O-C(R¹,R²)-A,
et E représente un électrophile qui est un aldéhyde, une cétone, un nitrile, une imine ou un alcyne,
et la réaction est effectuée dans un solvant aprotique.

2. Procédé conforme à la revendication 1, **caractérisé en ce que** l'alkyl-zinc masqué (B, C) est préparé par transmétallation d'un composé (F) comportant le groupe alkyle α,β-insaturé (A), d'après l'équation générale II : 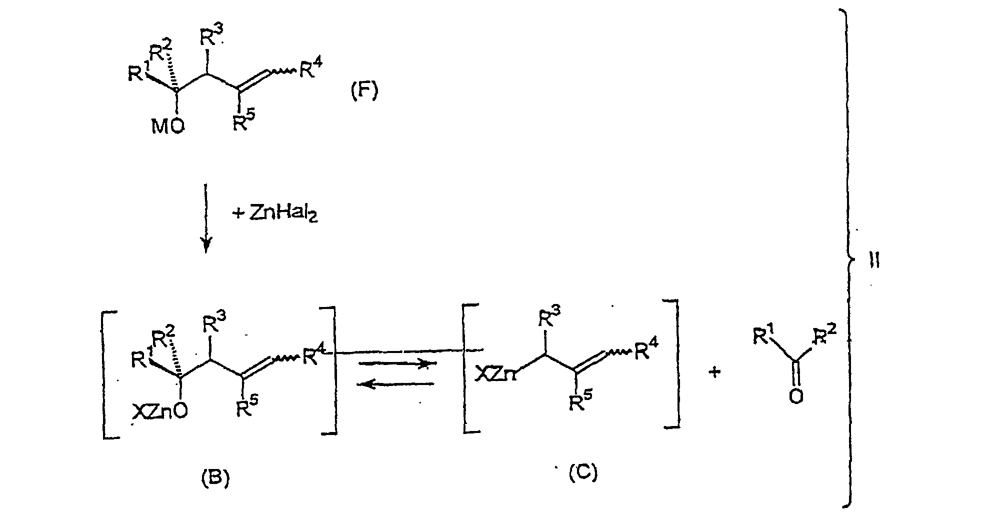 dans laquelle
M représente Li, Na, K ou MgX,
et Hal représente Cl, Br ou I,
et la réaction est effectuée dans un solvant aprotique.

3. Procédé conforme à la revendication 2, **caractérisé en ce que** l'on emploie le composé (F), le sel de zinc ZnHal₂ et l'électrophile (E) en les proportions molaires suivantes : 1/(0,1-1,5)/(0,8-3,0).

4. Procédé conforme à la revendication 2 ou 3, **caractérisé en ce que** l'on emploie le sel de zinc ZnHal₂ en une quantité de 0,1 à 0,5 mole par mole de composé de départ (F).

5. Procédé conforme à la revendication 1, **caractérisé en ce que** l'alkyl-zinc masqué (B, C) est préparé par métallation d'un composé (G) comportant le groupe alkyle α,β-insaturé (A) à l'aide d'un dialkyl-zinc ZnR₂, d'après l'équation générale III : 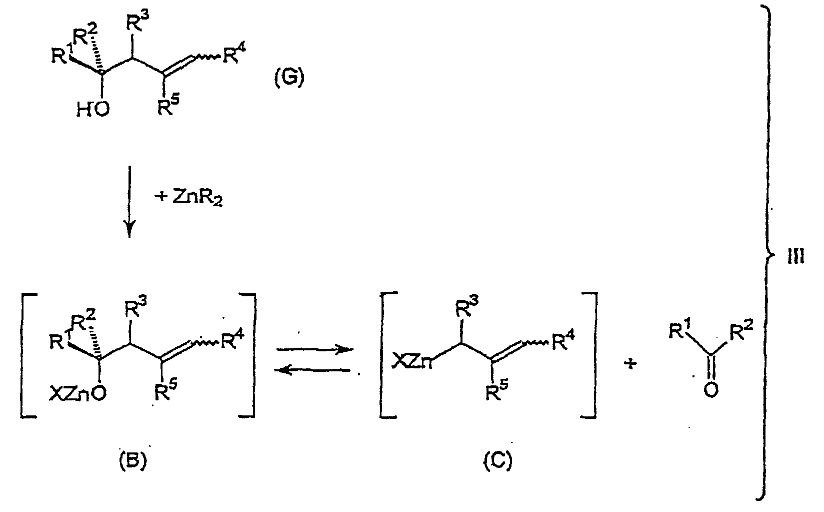 dans laquelle
R représente un groupe alkyle comportant 1 à 6 atomes de carbone,
et la réaction est effectuée dans un solvant aprotique.

6. Procédé conforme à l'une des revendications 1 à 5, **caractérisé en ce que** la préparation de l'alkyl-zinc masqué (B, C) et la réaction avec l'électrophile (E) ont lieu dans le même récipient.

7. Procédé conforme à l'une des revendications 1 à 6, **caractérisé en ce que** l'on emploie comme solvant aprotique un éther, un amide ou un hydrocarbure, ou un mélange de tels solvants.

8. Procédé conforme à l'une des revendications 1 à 7, **caractérisé en ce que** l'on emploie comme solvant aprotique de l'éther diéthylique, du tétrahydrofurane (THF), du 2-méthyl-tétrahydrofurane, de l'hexaméthylphosphotriamide (HMPT), de la N-méthyl-2-pyrrolidinone (NMP), du N,N-diméthylformamide (DMF), de la 1,3-diméthyl-tétrahydro-2(1H)-pyrimidinone (DMPU), du toluène, de l'hexane, de l'heptane, du cyclohexane ou un mélange de ces solvants.
